# EUROPEAN PATENT SPECIFICATION

(11) **EP 0 555 582 B1**
(45) Date of publication and mention of the grant of the patent: **04.06.1997**
(21) Application number: 92308786.0
(22) Date of filing: 25.09.1992
(51) Int. Cl.: A61M 25/00, B29C 65/70

(54) **Co-axial catheter**
Koaxial-Katheter
Cathéter coaxial

(30) Priority: 13.02.1992 CA 2061163
(43) Date of publication of application: 18.08.1993
(73) Proprietor: MED-PRO DESIGN, INC., Mississauga, Ontario L5G 2M3 (CA)
(72) Inventor: Martin, Geoffrey S., Mississauga, Ontario L5G 2M3 (CA)
(74) Representative: March, Gary Clifford

(56) References cited:
- EP-A- 0 346 613
- GB-A- 1 284 537

## Description

This invention relates to dual lumen catheters for use in haemodialysis treatments and more particularly to the structure of the proximal end of a dual lumen catheter having co-axial intake and return lumens.

Haemodialysis treatments have been developed since the early 1960s using a variety of combinations and arrangements of catheters. The earliest treatments were conducted using two needles in the same vein and this subsequently led to pioneer work done by Dr. Shaldon in England, who used two flexible catheters which could be left in place for limited periods. It was recognised by some practitioners that it would be preferable to use a single incision rather than to use two and this led to the development of techniques involving dual flow catheters. There are two basic types. The first to be attempted was a coaxial catheter with the intake lumen surrounding the return lumen. While this had advantages, there were some difficulties of manufacture. The other approach is to use side-by-side lumens either in individual tubes connected to one another or in a single tube divided by an interior septum so that the lumens are D-shaped. These structures also had advantages and disadvantages, the notable disadvantage being that, because the lumens are side-by-side, the intake openings must be in one side of the catheter. As a consequence of this, if the catheter were to attach itself to the wall of a vein due to suction applied to the intake lumen then, of course, the flow would stop. Medical staff then have to move the catheter by rotating it until blood again flows. This is a very delicate manipulation which is normally performed only by a qualified medical practitioner who must be available at all times in case the flow is discontinued.

The side-by-side structures have advantages in manufacture due to the fact that the two lumens can be created simultaneously in an extrusion. This had led to great activity in developing devices having side-by-side D-shaped lumens at the expense of co-axial structures. Nevertheless, due to the inherent disadvantages of the side-by-side structures, there has been renewed interest in developing suitable co-axial devices. This is primarily because the intake lumen can have openings in any part of the wall of the catheter. As a result, no matter where the catheter may rest against a vein, some of the intake openings remain patent. There is then less likelihood that the procedure must be serviced by a trained medical practitioner. An example of a co-axial device is found in British Patent Specification GB-A-1284537.

Catheters having side-by-side lumens can have intake tubes attached more or less in line with the lumens. By contrast, co-axial lumens have been used with the inner lumen connected to an axial tube and the outer tube to a fitting which projects transversely with respect to the axis of the catheter. It would be preferable to avoid this arrangement because it is inherent in such a structure that blood must pass along a path with a sudden change in direction and this can cause blood damage.

It is an object of the present invention to provide a co-axial catheter having inlet and outlet tubes projecting from the proximal end of the catheter generally in line with the axis of the catheter.

Accordingly, in one of its aspects, the invention provides a dual lumen co-axial catheter of the type having a main section extending axially and having proximal and distal ends and including an inner tube defining a return lumen, and an outer tube containing the inner tube and combining with the inner tube to define an annular intake lumen, the outer tube extending along a longitudinal axis and defining intake openings providing access into the intake lumen, intake and outlet tubes at the proximal end of the main section, and a junction coupling the tubes to the main section with the intake tube coupled for receiving blood from the intake lumen and the outlet tube coupled for delivering treated blood to the return lumen, the co-axial catheter being characterised by the inner and outer tubes being in contact inside the junction and the tubes leaving the junction to opposite sides of the axial direction with an angle of divergence of less than about 30 degrees.

In accordance with another aspect of the invention, a method of coupling inner and outer lumens of a co-axial dual lumen catheter to intake and outlet tubes at a proximal end of the catheter, the inner lumen being an inner tube and the outer lumen being the space between the inner tube and an outer tube which contains the inner tube loosely, the method characterised by providing a first mandrel having a cylindrical end part proportioned to fit closely inside the inner tube, and a first cylindrical portion angled with respect to the end part at an angle of less than about 15 degrees providing a second mandrel having a projection shaped to fit between the inner tube and the outer tube with the tubes in contact with one another, and a second cylindrical portion angled with respect to the projection at an angle of less than about 15 degrees placing the mandrels with the end part and the projection extending in parallel with the inner tube engaged on the end part and the projection engaged between the inner and outer tubes; engaging one of the intake and outlet tubes on each of the first and second cylindrical portions; molding a junction to contain parts of the outer tube, and intake and outlet tubes and removing the mandrels through the intake and outlet tubes.

These and other aspects of the invention will be better understood with reference to the drawings and the following description, in which:
Fig. 1 is an isometric view of a catheter according to a preferred embodiment of the invention;
Fig. 2 is a sectional view of Fig. 1 drawn to a larger scale; and
Fig. 3 is a partially sectional view showing a junction at the proximal end of the catheter and demonstrating both the structure and a method of making the catheter with this junction.

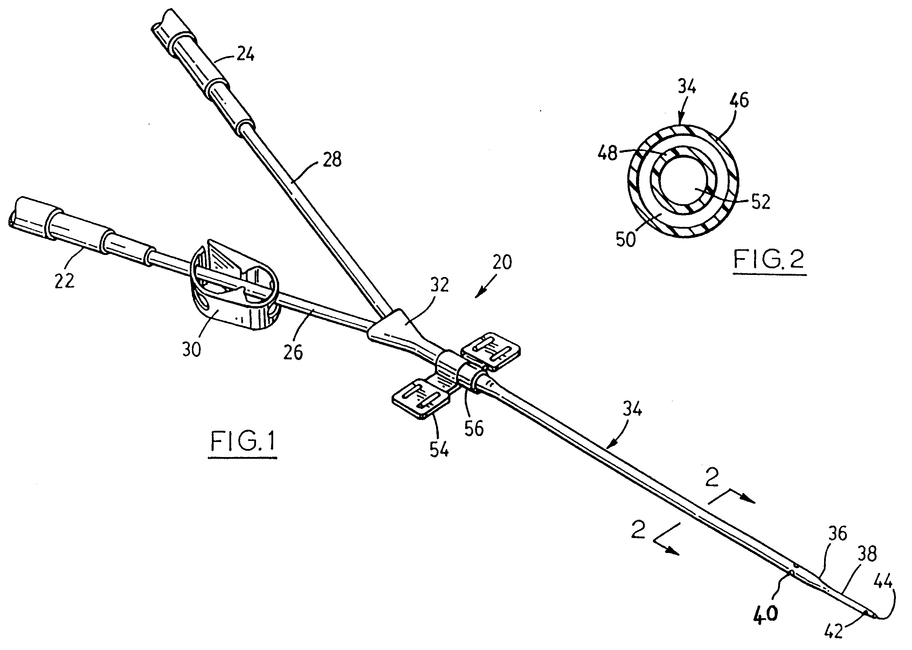

Reference is made firstly to Fig. 1 which illustrates a catheter designated generally by the numeral 20 and useful for withdrawing blood through an intake 22 and returning treated blood through an outlet 24. The intake and outlet are connected by flexible tubes 26, 28 which can be clamped using conventional devices such as device 30 shown in tube 26. The tubes meet at a junction 32 at the proximal end of a main section 34 which terminates at its distal end in a transition portion 36 leading to a tip section 38. Blood is withdrawn through side openings 40 and returns through further side openings 42 and end opening 44.

As seen in Fig. 2, the main section 34 includes an outer tube 46 containing an inner tube 48. The openings 40, shown in Fig. 1, supply blood to an intake lumen 50 formed between the tubes 46, 48 and blood returns by a return lumen 52 contained within the inner tube 48. The junction 32 at the proximal end of the main section 34 connects the main portion to the tubes 26, 28 (as will be explained) and the catheter is completed by provision of a wing structure 54 used to attached the catheter in place in conventional fashion. It is preferable that the wing structure be rotatable on the catheter and provision is made for this with location provided by a sleeve 56 which prevents movement of the wing structure longitudinally relative to the catheter.

The side openings 40 and 42 are typical of openings that can be provided around the periphery of the catheter to ensure flow into and out of the catheter from anywhere about the catheter. Consequently, if the catheter should be positioned against the wall of a vein, other openings will take over and provide the essential flow.

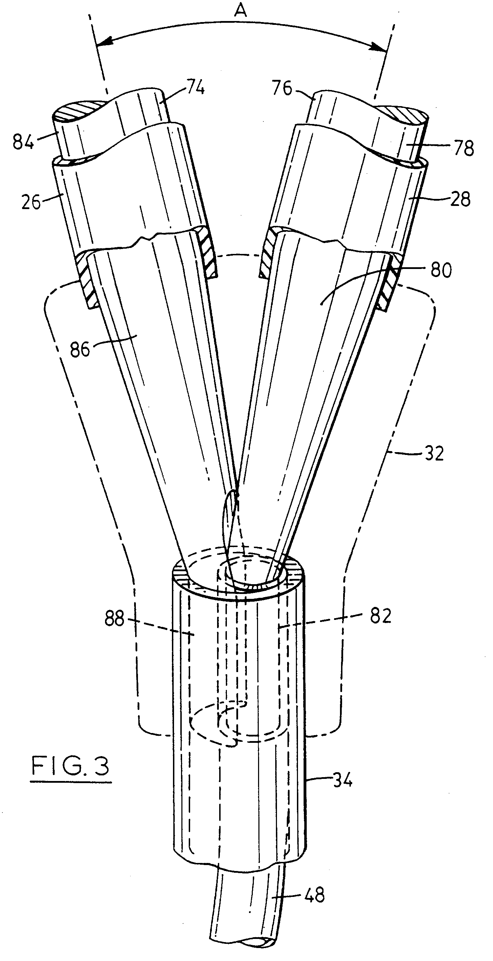

Reference is next made to Fig. 3 which illustrates the details of the junction 32, and in particular the method of manufacturing this junction. As seen in Fig. 3, the junction is prepared by first positioning a proximal end of the main section 34 in a mold (not shown) which is to create the junction 32 by injection molding using conventional techniques. The main section is positioned using first and second mandrels 74, 76. The mandrel 76 has a cylindrical portion 78 blending into a converging generally conical pattern 80, which in turn blends into a cylindrical end part 82 angled with respect to the portion 80. The part 82 fits closely inside a proximal end of the inner tube 48 and this tube is maintained in a position in engagement with the outer tube 46 by the mandrels 74, 76.

The mandrel 74 has an outer cylindrical portion 84 which blends into a converging and generally conical portion 86 ending at a projection 88. This projection has a generally U-shaped configuration (as will be explained) and is angled with respect to the conical portion 86.

The projection 88 on the end of the mandrel 74 is shaped to fit the space provided when the inner tube 48 is held against the inner surface of the outer tube 46. As a result it has a generally U-shaped configuration. The angular offsets of the projection 88 and the end part 82 of lumen 76 result in the projection and end part 82 extending in parallel axially with respect to the main section 34. The cylindrical portions 78 and 84 diverge sufficiently with respect to the axial main section that the ends of the respective intake and outlet tubes 26, 28 can be accommodated on the mandrels.

Once the assembly shown in Fig. 3 has been completed, the mold is closed and injection takes place to form the junction 32. The material is preferably polyurethane although other materials can be used provided that the usual requirements of compatibility, etc., are met.

The mandrels are removed, and because there is some flexibility in the material, the mandrels can be pulled out without causing any damage.

The structure shown in Fig. 3 has particular significance in the resulting flow through the catheter. Unlike previous co-axial catheters, the flow tends to remain linear due to the fact that the intake and return tubes 26, 28 are generally in line with the main section 34. Previously, one of these tubes was in line and the other was connected through the side of the main section so that the flow must pass through a significant angle which in some instances approached 90 degrees. This is most undesirable because any changes in direction of this kind will result in turbulence in the blood flow with potential for damage to the blood. It is well established that pressure fluctuations in blood flow paths should be minimized, and this structure tends to limit such variations.

It is also significant that the resulting structure presents a smooth continuous internal surface to blood flow which again is desirable.

The angle shown as "A" in Fig. 3 indicates the divergence between the tubes 26, 28 as they leave the junction 32. Because of the construction, it is possible to maintain this angle in the order of 15 to 20 degrees and is readily maintained below 30 degrees. As a result, the flow into and out of the catheter is essentially axial with reference to the main section 34 at all times. This is because the angle of the tubes 26, 28 with reference to the axis of the main section is half of the range up to 30 degrees.

The embodiment described is representative of the invention and other embodiments and variations are within the scope of the invention as claimed.

## Claims

1. A dual lumen co-axial catheter of the type having a main section (34) extending axially and having proximal and distal ends and including an inner tube (48) defining a return lumen (52), and an outer tube (46) containing the inner tube and combining with the inner tube to define an annular intake lumen (50), the outer tube extending along a longitudinal axis and defining intake openings (40) providing access into the intake lumen, intake and outlet tubes (22, 24) at the proximal end of the main section, and a junction (32) coupling the tubes to the main section with the intake tube coupled for receiving blood from the intake lumen and the outlet tube coupled for delivering treated blood to the return lumen, the co-axial catheter being characterised by the inner and outer tubes (48, 46) being in contact inside the junction and the tubes (22, 24) leaving the junction to opposite sides of the axial direction with an angle of divergence of less than about 30 degrees.

2. A catheter as recited in claim 1 characterised in that the angle of divergence is about 15 to 20 degrees.

3. A catheter as recited in claims 1 or 2 characterised in that the inner tube, outer tube and tip section are round in cross-section.

4. A method of coupling inner and outer lumens (52, 50) of a co-axial dual lumen catheter (20) to intake and outlet tubes (22, 24) at a proximal end of the catheter, the inner lumen being an inner tube (48) and the outer lumen being the space between the inner tube and an outer tube (46) which contains the inner tube loosely, the method characterised by providing a first mandrel (76) having a cylindrical end part (82) proportioned to fit closely inside the inner tube, and a first cylindrical portion (78) angled with respect to the end part at an angle of less than about 15 degrees providing a second mandrel (74) having a projection (88) shaped to fit between the inner tube and the outer tube with the tubes in contact with one another, and a second cylindrical portion (84) angled with respect to the projection at an angle of less than about 15 degrees placing the mandrels with the end part (82) and the projection (88) extending in parallel with the inner tube (48) engaged on the end part (82) and the projection (88) engaged between the inner and outer tubes (48, 46); engaging one of the intake and outlet tubes (22, 24) on each of the first and second cylindrical portions (78, 84); molding a junction to contain parts of the outer tube, and intake and outlet tubes; and removing the mandrels through the intake and outlet tubes.

## Patentansprüche

1. Ein zweilumiger koaxialer Katheter des Typs, der einen Hauptabschnitt (34) aufweist, der axial verläuft und proximale und distale Enden aufweist und einen Innenschlauch (48) einschließt, der ein Rücklauflumen (52) definiert, und einen Außenschlauch (46), der den Innenschlauch enthält und in Zusammenwirken mit dem Innenschlauch ein ringförmiges Einlauflumen (50) definiert, wobei der Außenschlauch entlang einer Längsachse verläuft und Einlaßöffnungen (40) definiert, die Zugang zum Einlauflumen bereitstellen, Einlaß- und Auslaßschläuche (22, 24) am proximalen Ende des Hauptabschnittes und ein Anschlußstück (32), das die Schläuche an den Hauptabschnitt ankoppelt, wobei der Einlaßschlauch so angekoppelt ist, daß er Blut aus dem Einlauflumen aufnimmt, und der Auslaßschlauch so angekoppelt ist, daß er behandeltes Blut zum Rücklauflumen zuführt, wobei der koaxiale Katheter dadurch gekennzeichnet ist, daß die Innen- und Außenschläuche (48, 46) innerhalb des Auschlußstückes in Kontakt miteinander stehen und die Schläuche (22, 24) zu entgegengesetzten Seiten der axialen Richtung mit einem Divergenzwinkel von weniger als etwa 30 Grad aus dem Anschlußstück austreten.

2. Ein Katheter nach Anspruch 1, dadurch gekennzeichnet, daß der Divergenzwinkel etwa 15 bis 20 Grad beträgt.

3. Ein Katheter nach den Ansprüchen 1 oder 2, dadurch gekennzeichnet, daß der Innenschlauch, der Außenschlauch und der Spitzenabschnitt im Querschnitt rund sind.

4. Ein Verfahren zum Verbinden von Innen- und Außenlumina (52, 50) eines koaxialen zweilumigen Katheters (20) mit Einlaß- und Auslaßschläuchen (22, 24) an einem proximalen Ende des Katheters, wobei das Innenlumen ein Innenschlauch (48) ist und das Außenlumen der Zwischenraum zwischen dem Innenschlauch und einem Außenschlauch (46) ist, der den Innenschlauch locker enthält, wobei das Verfahren dadurch gekennzeichnet ist, daß ein erster Dorn (76) mit einem zylindrischen Endstück (82) bereitgestellt wird, der so dimensioniert ist, daß er eng in den Innenschlauch hineinpaßt, und einem zylindrischen Abschnitt (78), der in Bezug auf das Endstück mit einem Winkel von weniger als etwa 15 Grad abgewinkelt ist, ein zweiter Dorn (74) mit einem Vorsprung (88) bereitgestellt wird, der so ausgebildet ist, daß er zwischen den Innenschlauch und den Außenschlauch paßt, wobei die Schläuche in Kontakt miteinander stehen, und einem zweiten zylindrischen Abschnitt (84), der in Bezug auf den Vorsprung mit einem Winkel von weniger als etwa 15 Grad abgewinkelt ist, wobei die Dorne so angeordnet sind, daß das Endstück (82) und der Vorsprung (88) parallel mit dem Innenschlauch (48) verlaufen, der mit dem Endstück (82), und dem Vorsprung (88) in Eingriff steht, der zwischen dem Innen- und Außenschlauch (48, 46) sitzt, verläuft; einen von den Einlaß- und Auslaßschläuchen (22, 24) auf jeden der ersten und zweiten zylindrischen Abschnitte (78, 84) geschoben wird; ein Anschlußstück ausgeformt wird, so daß es Teile des Außenschlauches und der Einlaß- und Auslaßschläuche enthält; und die Dorne durch die Einlaß- und Auslaßschläuche entfernt werden.

## Revendications

1. Cathéter co-axial à conduit double du type comprenant une partie principale (34) s'étendant axialement et comprenant des extrémités proximale et distale et incluant un tube interne (48) définissant un conduit de retour (52), et un tube externe (46) contenant le tube interne et se combinant au tube interne pour définir un conduit d'admission annulaire (50), le tube externe s'étendant le long d'un axe longitudinal et définissant des ouvertures d'admission (40) fournissant un accès dans le conduit d'admission, des tubes d'admission et d'évacuation (22,24) à l'extrémité proximale de la partie principale, et une jonction (32) couplant les tubes à la partie principale, le tube d'admission étant couplé pour recevoir du sang du conduit d'admission et le tube d'évacuation couplé pour délivrer du sang traité au conduit de retour, le cathéter co-axial étant caractérisé en ce que les tubes d'admission et d'évacuation (48,46) sont en contact à l'intérieur la jonction et en ce que les tubes (22,24) quittent la jonction vers des côtés opposés de la direction axiale avec un angle de divergence de moins d'environ 30 degrés.

2. Cathéter selon la revendication 1, caractérisé en ce que l'angle de divergence est d'environ 15 à 20 degrés.

3. Cathéter selon l'une des revendication 1 et 2, caractérisé en ce que le tube interne, le tube externe et une partie d'extrémité sont ronds en section transversale.

4. Procédé de couplage des conduits internes et externes (52,50) d'un cathéter co-axial à conduit double (20) à des tubes d'admission et d'évacuation (22,24), le conduit interne étant un tube interne (48) et le conduit externe étant l'espace entre le tube interne et le tube externe (46) qui contient de manière flottante le tube interne, procédé caractérisé en ce qu'on prévoit un premier mandrin (76) ayant une première partie cylindrique d'extrémité (82) proportionnée pour s'ajuster étroitement à l'intérieur du tube interne, et une première portion cylindrique (78) inclinée par rapport à la partie d'extrémité selon un angle de moins d'environ 15 degrés, on prévoit un second mandrin (74) ayant une saillie (88) de forme adéquate pour s'ajuster entre le tube interne et le tube externe en contact l'un avec l'autre, et une seconde portion cylindrique (84) inclinée par rapport à la saillie selon un angle de moins d'environ 15 degrés, on place les mandrins de telle sorte que la partie d'extrémité (82) et la saillie (88) s'étendent en parallèle, le tube interne (48) s'engageant sur la partie d'extrémité (82) et la saillie (88) s'engageant entre les tubes interne et externe (48,46); on engage l'un des tubes d'admission et d'évacuation (22,24) sur chacune des première et seconde parties cylindriques (78,84); on moule une jonction pour contenir des parties du tube externe et des tubes d'admission et d'évacuation; et on retire les mandrins à travers les tubes d'admission et d'évacuation.
